(19) Europäisches Patentamt
European Patent Office
Office européen des brevets

(11) **EP 3 319 026 A1**

(12) **EUROPEAN PATENT APPLICATION**

(43) Date of publication:
**09.05.2018 Bulletin 2018/19**

(51) Int Cl.:
*G06Q 10/08* (2012.01)    *G01N 35/00* (2006.01)

(21) Application number: **16197229.4**

(22) Date of filing: **04.11.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicants:
• **F. Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**
• **Roche Diagnostics GmbH
68305 Mannheim (DE)**
Designated Contracting States:
**DE**

(72) Inventors:
• **BAERISWYL, Thomas
6010 Kriens (CH)**
• **EGAN, David
8044 Zürich (CH)**

(74) Representative: **Gyenge, Zoltán
Roche Diagnostics International AG
Patents
Forrenstrasse 2
6343 Rotkreuz (CH)**

Remarks:
A request for correction of claim 1 has been filed pursuant to Rule 139 EPC. A decision on the request will be taken during the proceedings before the Examining Division (Guidelines for Examination in the EPO, A-V, 3.).

(54) **INVENTORY ALERT SYSTEM FOR LABORATORIES**

(57)    Disclosed herein is a system and computer implemented method for detecting extraordinary demand of a consumable(s) - for processing biological samples by laboratory instrument(s) in a plurality of laboratories - based on exceptional deviations from usual demand levels of trigger laboratories in the proximity of a target laboratory.

FIG. 1

EP 3 319 026 A1

**Description**

TECHNICAL FIELD

The present disclosure relates to an inventory alert system for laboratories, in particular laboratories comprising at least one laboratory instrument configured to carry out at least one processing step of a biological sample, in particular a laboratory test. The present disclosure further relates to a computer implemented method for alerting laboratories of consumable demand deviation.

BACKGROUND

In vitro diagnostic testing has a major effect on clinical decisions, providing physicians with pivotal information. Particularly, there is great emphasis on providing quick and accurate test results in critical care settings.

Diagnostic testing makes use of various consumables such as reagents, quality control material such as positive and negative controls, calibrator material, microplates/ microwell plates, measurement cuvettes, sample tubes, pipetting tips, etc. In order to be able to carry out laboratory tests, it is therefore of utmost importance that laboratories have all required consumables available.

Existing inventory management systems for diagnostic laboratories have fixed configured values for the maximum amount of each consumable that is ordered and/or stored in a laboratory (stock). The customer enters a value, and the system uses this value until the system is re-configured. Orders of consumables are based on the current stock and a (usually static) maximum amount of the consumable to be stocked, an order being triggered when the current stock drops below a set threshold value.

Widespread occurrence of an infectious disease or even an increased likelihood of the same in a region at a particular time cause laboratories to perform more of a specific laboratory measurement of a biological sample (laboratory test) than they would normally expect based on historical data or normal/predicable seasonal fluctuations. Therefore, there is a risk that laboratories run out of consumables during periods of peak consumption which would lead to delays in obtaining laboratory measurement results.

In order to reduce this risk of running out of consumables, currently laboratories increase their stock to cover even more than the expected need for a particular consumable, in an attempt to buffer unpredictable situations.

However, storage space needs to be used to its utmost efficiency as it is coupled with both set-up and recurring costs. This applies even more in case of storage of consumables such as reagents which need to be kept in a defined temperature range, e.g. in a refrigerator. Furthermore, a stock that constantly exceeds the demand/usage (by a safety margin) inevitably leads to waste, as most of the consumables required for laboratory tests have a strict expiration date.

Therefore there is a need for an inventory system which ensures that all consumable(s) are always available for all required laboratory tests while avoiding use of unnecessary storage space or waste consumables by too large of a reserve stock of consumables, even when demand deviates from normal/expected patterns of demand.

SUMMARY

Disclosed herein is a system and computer implemented method for detecting extraordinary demand of a consumable(s) - for processing biological samples by laboratory instrument(s) - based on exceptional deviations from usual demand levels of trigger laboratories in the proximity of a target laboratory.

According to embodiments herein disclosed, the inventory alert system for laboratories comprises a plurality of laboratories and a control system communicatively connected thereto. The plurality of laboratories comprise at least one triggering laboratory and at least one target laboratory, while each laboratory comprising at least one laboratory instrument configured to carry out at least one processing step of a biological sample. At least one consumable is required to carry out the processing step of a biological sample.

The inventory alert system is configured in a first step to determine a usual demand level $T$ of the consumable required to carry out the processing step of a biological sample by the corresponding triggering laboratory.

In a further step, the inventory alert system processes actual usage data $X$ of the consumable required to carry out the processing step of a biological sample by the respective triggering laboratory. Based on this data, the inventory alert system determines a deviation $D$ of the actual usage data $X$ from the usual demand level $T$ of the corresponding triggering laboratory.

An alert triggering factor $F$ is then calculated for each deviation $D$ that exceeds an exceptional deviation threshold. These deviations are called exceptional deviations. Deviations below the exceptional deviation threshold are considered normal fluctuations in the actual usage of the consumables and are not considered for triggering any alerts.

Target laboratory(s) are alerted when the aggregation of each alert triggering factor $F$ corresponding to each triggering laboratory in the proximity of the target laboratory exceeds an alert threshold $AT$.

According to embodiments herein disclosed, the inventory alert system respectively the disclosed alert method are advantageous on multiple levels of configurability and adaptability are enabled thereby:

-     by means of an exceptional deviation threshold, normal consumable demand fluctuations are filtered out in a manner specific to each triggering laboratory;

- by means of a proximity function - and its weighting(s), the relevance (or influence) of each triggering laboratory is fine tuned to the specific target laboratory; and
- by setting an appropriate alert threshold of aggregated triggering factors, false triggers due to alert trigger factors of low relevance can be filtered out.

In particular embodiments of the disclosed system and method, the usual demand level of the consumable required to carry out the processing step of a biological sample is determined based on an average and/or median number of conducted and/or ordered processing step(s) per defined period corresponding to the respective triggering laboratory and/or determined based on historical data comprising one or more of: a seasonal variation; a schedule-based variation; an event-linked variation and a trend associated variation; geographically influenced variation.

In particular embodiments of the disclosed system and method, the alert triggering factor is a function of one or more of: the usual demand level, the exceptional deviation and the proximity P of the triggering laboratory and the target laboratory.

In particular embodiments of the disclosed system and method, the proximity P is a function, in particular a weighted function.

In particular embodiments of the disclosed system and method, alerting the target laboratory comprises calculation of an expected demand deviation of the consumable for the target laboratory, the expected demand deviation being a function of the usual demand level T of the target laboratory and/or a function of each alert triggering factor F corresponding to each triggering laboratory in the proximity P of the target laboratory and/or a function of the deviation D of each triggering lab.

In particular embodiments of the disclosed system and method, alerting the target laboratory comprises generation of an order sheet of the corresponding consumable based on the expected demand deviation of the consumable for the target laboratory and the actual inventory of the consumable in the target laboratory and/or automatically ordering the corresponding consumable based on the order sheet for target laboratory.

In particular embodiments of the disclosed system and method, the at least one laboratory instrument comprises at least one analytical instrument and wherein the at least one processing step of a biological sample comprises at least one diagnostic test of the biological sample for obtaining a measurement value from biological sample.

In particular embodiments of the disclosed system and method, the at least one laboratory instrument comprises at least one pre-analytical instrument and wherein the at least one processing step of a biological sample comprises the centrifugation, aliquotation, analyte isolation of an analyte from the biological sample.

BRIEF DESCRIPTION OF DRAWINGS

Further characteristics and advantages of the disclosed method / device /system will in the following be described in detail by means of the description and by making reference to the drawings, which show:

Fig. 1 A schematic diagram of an embodiment of the inventory alert system comprising a plurality of laboratories communicatively connected with a control unit by means of a communication network;

Fig. 2 A flowchart depicting the disclosed method for alerting laboratories, respectively the processing steps the inventory alert system is configured to carry out;

Fig. 3 A chart showing actual usage data X; usual demand level T of the consumable as well as exceptional deviation threshold EDT of a particular consumable required to carry out a processing step of biological sample by a laboratory;

Fig. 4 An example of geographic distribution of a plurality of laboratories of the disclosed inventory alert system, the plurality of laboratories comprising at least one triggering laboratory and at least one target laboratory;

Fig. 5 A flowchart depicting a further embodiment of the disclosed method for alerting laboratories, respectively the processing steps the inventory alert system is configured to carry out, further comprising the calculation of an expected demand deviation of the consumable for the target laboratory;

Fig. 6 A flowchart depicting a further embodiment of the disclosed method for alerting laboratories, respectively the processing steps the inventory alert system is configured to carry out, further comprising generation of an order sheet or automatic order of the corresponding consumable based on the expected demand deviation of the consumable for the target laboratory

Fig. 7 A schematic diagram of a further embodiment of the inventory alert system, further comprising an input device communicatively connected to the control unit.

Note: The figures are not drawn to scale, are provided as illustration only, and serve only for better understanding, not for defining the scope of the invention. No limitations of any features of the invention should be inferred from these figures.

DETAILED DESCRIPTION

Certain terms will be used in this patent application, the formulation of which should not be interpreted to be limited by the specific term chosen, but as to relate to the general concept behind the specific term.

As used herein, the terms 'comprises,' 'comprising,' 'includes,' 'including,' 'has,' 'having' or any other variation thereof, are intended to cover a non-exclusive inclusion of features.

An 'analysis system' as used herein comprises a control unit operatively coupled to one or more analytical; pre- and post-analytical devices wherein the control unit is operable to control the devices. In addition, the control unit may be operable to evaluate and/or process gathered analysis data, to control the loading, storing and/or unloading of samples and/or consumables to and/or from any one of the devices, to initialize an analysis or hardware or software operations of the analysis system used for preparing the samples, sample tubes or reagents for said analysis and the like.

The term 'analyte' is a component of a sample to be analyzed, e.g. molecules of various sizes, ions, proteins, metabolites and the like. Information gathered on an analyte may be used to evaluate the impact of the administration of drugs on the organism or on particular tissues or to make a diagnosis. Thus 'analyte' is a general term for substances for which information about presence and/or concentration is intended. Examples of analytes are e.g. glucose, coagulation parameters, endogenic proteins (e.g. proteins released from the heart muscle), metabolites, nucleic acids and so on.

The term 'analytical data' as used herein encompasses any data that is descriptive of a result of a measurement of a biological sample. In case of a calibration the analytical data comprises the calibration result, i.e. calibration data. In particular, the analytical data comprises an identifier of the sample for which the analysis has been performed and data being descriptive of a result of the analysis, such as measurement data.

The term 'laboratory instrument' as used herein encompasses any apparatus or apparatus component operable to execute one or more processing steps / workflow steps on one or more biological samples and/or one or more reagents. The expression 'processing steps' thereby refers to physically executed processing steps such as centrifugation, aliquotation, analyte isolation, sample analysis and the like. The term 'instrument' covers pre-analytical instruments, post-analytical instruments and also analytical instruments.

The term 'analyzer' / 'analytical instrument' as used herein encompasses any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectroscopy of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit or consumable loading and unloading unit. The analyzer may comprise a process and detection system whose workflow is optimized for certain types of analysis. Examples of such analyzer are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'point of care analyzer' as used herein encompasses any analyzer used in a point of care environment, such as (but not limited to) blood glucose testing, coagulation testing, blood gas and electrolytes analysis, urinalysis, cardiac markers analysis, hemoglobin diagnostics, infectious disease testing and cholesterol screening. Results may be viewed directly on the POC analyzer(s) or may be sent to the POC system and displayed in a Laboratory Middleware or a Laboratory Information System (LIS) with central lab results, or alongside imaging results in a Hospital Information System (HIS).

The term 'analysis or 'analytical test' as used herein encompasses a laboratory procedure characterizing a parameter of a biological sample for qualitatively assessing or quantitatively measuring the presence or amount or the functional activity of an analyte.

The term 'pre-analytical instrument' as used herein encompasses any apparatus or apparatus component that is configured to perform one or more pre-analytical processing steps / workflow steps comprising - but not limited to - centrifugation, analyte isolation, resuspension (e.g. by mixing or vortexing), capping, decapping, recapping, sorting, tube type identification, sample quality determination and/or aliquotation steps. Said processing steps may also comprise adding chemicals or buffers to a sample, concentrating a sample, incubating a sample, and the like.

The term 'post-analytical instrument' as used herein encompasses any apparatus or apparatus component that is configured to perform one or more post-analytical processing steps / workflow steps comprising - but not limited to - sample unloading, transport, recapping, decapping, temporary storage/ buffering, archiving (refrigerated or not), retrieval and/ or disposal.

The term 'communication network' as used herein encompasses any type of wireless network, such as a WIFI, GSM, UMTS or other wireless digital network or a cable based network, such as Ethernet or the like. In particular, the communication network can implement the Internet protocol (IP). For example, the communication network comprises a combination of cable-based and wireless networks.

The term 'control unit' as used herein encompasses any physical or virtual data processing device. In some embodiments, the control unit might be integral with a data management unit, may be comprised by a server computer and/or be part of one laboratory instrument or even distributed across multiple instruments of the laboratory system. The control unit may, for instance, be embodied as a programmable logic controller running a computer-readable program provided with instructions to perform operations. A "data management unit" is a computing unit for storing and managing data. This may involve data relating to consumable(s) required to carry out processing step(s) on biological samples. The data management unit may be connected to an LIS (laboratory information system) and/or an HIS (hospital information system). The data management unit (DMU) can be a unit within or co-located with an automated system. It may be part of the control unit. Alternatively, the DMU may be a unit remotely located. For instance, it may be embodied in a computer connected via a communication network.

The terms 'sample', 'patient sample' and 'biological sample' refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

The term 'user interface' as used herein encompasses any suitable piece of software and/or hardware for interactions between an operator and a machine, including but not limited to a graphical user interface for receiving as input a command from an operator and also to provide feedback and convey information thereto. Also, a system /device may expose several user interfaces to serve different kinds of users/ operators.

The inventory alert system 1 for laboratories respectively the computer implemented method for alerting laboratories shall be now described with reference to particular embodiments thereof.

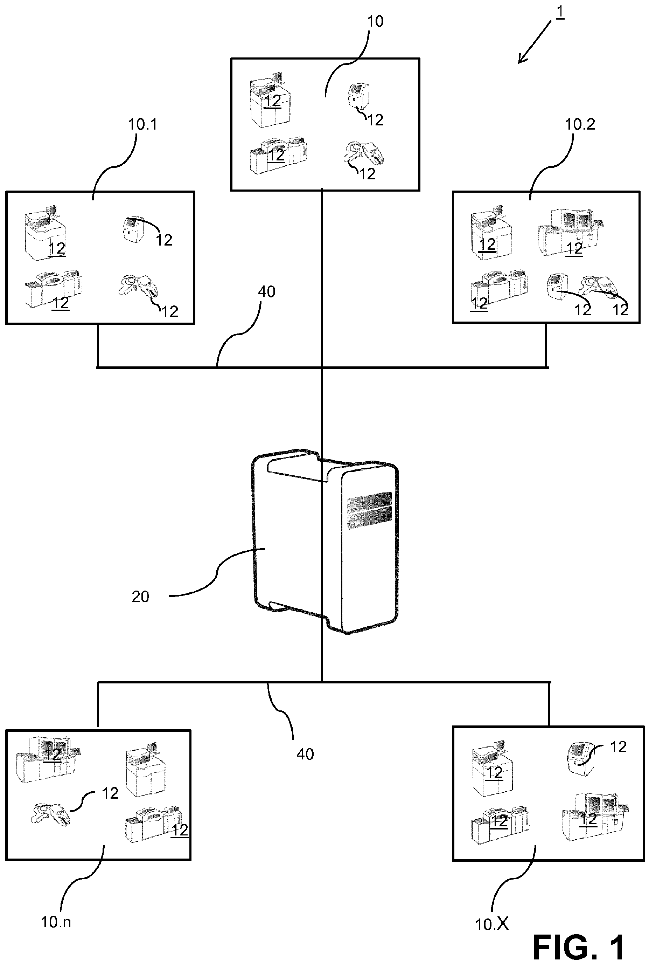

As illustrated on figure 1, the inventory alert system 1 for laboratories comprises a plurality of laboratories 10 communicatively connected with a control unit 20 by means of a communication network 40. The laboratories 10 may be located in different geographical locations and/or in different areas of one particular location, such as various labs of the same hospital, diagnostic center, etc.

Each laboratory 10 of the plurality of laboratories 10 comprises at least one laboratory instrument 12 configured to carry out at least one processing step of a biological sample, a consumable being required to carry out the processing step. The at least one laboratory instrument 12 may be one or more of a pre-analytical instrument; analytical instrument and/or post-analytical instrument. Correspondingly the at least one processing step of a biological sample comprises one or more of:

- pre-analytical processing step(s), comprising - but not limited to - centrifugation, separation/ isolation, resuspension (e.g. by mixing or vortexing), capping, decapping, recapping, sorting, tube type identification, sample quality determination and/or aliquotation steps;
- diagnostic test(s) of the biological sample for obtaining a measurement value from biological sample; and/or
- post-analytical processing steps, comprising - but not limited to - sample unloading, transport, recapping, decapping, temporary storage/ buffering, archiving (refrigerated or not), retrieval and/ or disposal.

Corresponding to the processing step of a biological sample, the at least one consumable being required to carry out the processing step comprises - but not limited to - one or more of: reagents; diluents; liquid handling disposables such as pipetting tips; reaction vessels, such as cuvettes, sample plates, microwell plates, etc.; sample tubes; sample tube racks.

The plurality of laboratories 10 comprise at least one laboratory which is a triggering laboratory 10.1-10.n and at least one laboratory which is a target laboratory 10.X. It shall be noted that the same laboratory 10 may be both a triggering laboratory 10.1-10.n and target laboratory 10.X at different points in time.

The at least one triggering laboratory 10.1-10.n and at least one target laboratory 10.X a communicatively connected to each of the plurality of laboratories 10 by means of a communication network 40, which may be a wired or wireless communication network or a combination thereof.

Figure 1 shows one embodiment of the disclosed system 1 wherein the control unit 20 is a stand-alone, dedicated server computer. Nevertheless, according to various embodiments (not shown on the figures) the control unit 20 may be a virtual processing device comprised by a multi-purpose server computer and/or be part of one laboratory instrument or even distributed across multiple instruments of the laboratory system.

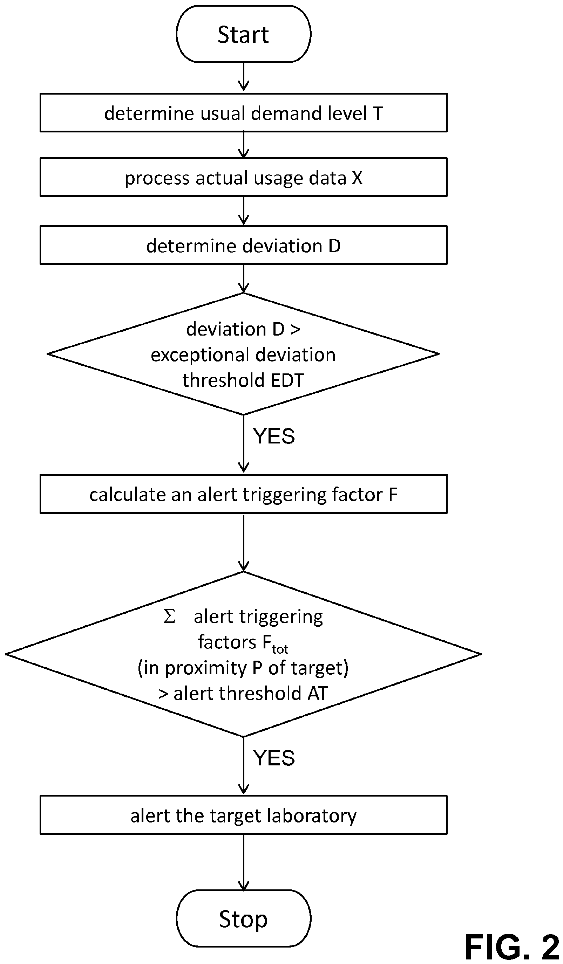

Turning now to figure 2, the steps of the disclosed method for alerting laboratories shall be described - the method being implemented by a computer system, in particular by the inventory alert system 1 configured to carry out such method steps.

In a first step, the inventory alert system 1 is configured to determine a usual demand level T of the consumable required to carry out the processing step by the corresponding triggering laboratory 10.1-10.n.

According to a first embodiment, the usual demand level T is determined by the respective triggering laboratory 10.1-10.n itself. In such embodiments, the triggering laboratory 10.1-10.n is configured to transmit the usual demand level T to the control unit 20 via the communication network 40.

Alternatively or additionally, according to a further embodiment, the usual demand level T is determined by the control unit 20 based on actual usage data received from the corresponding triggering laboratory 10.1-10.n.

The usual demand level T represents the expected consumption rate of corresponding consumables of a laboratory 10 and is determined based on an average and/or median number of conducted and/or ordered processing step(s) per defined period corresponding to the respective triggering laboratory 10.1-10.n and/or determined based on historical data comprising one or more of: a seasonal variation; a schedule-based variation; an event-linked variation and a trend associated variation; geographically influenced variation.

A seasonal fluctuation is, in the context of the present disclosure, a fluctuation of the consumption of a consumable required to carry out a processing step on a biological sample which is influenced by calendar seasons, e.g. flu season, when certain diagnostic tests are carried out more frequently than for example in summer.

A schedule-based fluctuation is, in the context of the present disclosure, a fluctuation of the consumption of a consumable required to carry out a processing step on a biological sample which is influenced by a schedule of, e.g. weekday vs. weekend, day shift/ night shift.

An event-linked fluctuation is, in the context of the present disclosure, a fluctuation of the consumption of a consumable required to carry out a processing step on a biological sample which is influenced by an particular event, e.g. a major event, such as Olympic games with a known/ expected contamination risk leading to increased demand for a test (e.g. increased Zika Virus testing around the world following the 2016 Olympic Games in Rio de Janeiro). A trend associated fluctuation is, in the context of the present disclosure, a fluctuation of the consumption of a consumable required to carry out a processing step on a biological sample which is influenced by a long term tendency to perform more and more test of a type. e.g. due to aging population; due to increased % of population with diabetes, etc.

Geographically influenced fluctuation is, in the context of the present disclosure, a fluctuation of the consumption of a consumable required to carry out a processing step on a biological sample which is influenced by the geographical location of the respective triggering laboratory 10.1-10.n, e.g. increased Ebola infections in Sub-Saharan Afrika, increased HIV test in 3rd world countries, etc.

In a following step, the inventory alert system 1 is configured to process actual usage data X of the consumable required to carry out the processing step by the corresponding triggering laboratory 10.1-10.n in order to determine a deviation D of the actual usage data X from the usual demand level T of the corresponding triggering laboratory 10.1-10.n.

According to a first embodiment, the processing of the actual usage data X and/or determination of the deviation D of the actual usage data X from the usual demand level T is performed by the respective triggering laboratory 10.1-10.n itself. In such embodiments, the triggering laboratory 10.1-10.n is configured to transmit the deviation D to the control unit 20 via the communication network 40.

Alternatively or additionally, according to a further embodiment, the processing of the actual usage data X and/or determination of the deviation D of the actual usage data X from the usual demand level T is performed by the control unit 20 based on actual usage data X received from the respective triggering laboratory 10.1-10.n via the communication network 40. According to further embodiments of the disclosed system/ method, the usual demand level T and/or actual usage data X are anonymized to protect the privacy of the plurality of triggering laboratories 10.1-10.n.

After the deviation D is determined, it is compared to an exceptional deviation threshold EDT. If the deviation D is below the exceptional deviation threshold EDT, the deviation D is considered as a normal- fluctuation of consumable use and therefore is filtered out, namely such deviations are excluded from further consideration. According to embodiments disclosed, the exceptional deviation threshold EDT is set based on historical data, e.g. as a percentage of the usual demand level T of the consumable; an absolute value; etc. The exceptional deviation threshold EDT is set such as to filter out the normal fluctuation of consumable use to avoid "false alarms" but at the same ensuring that exceptional deviations are reliably detected.

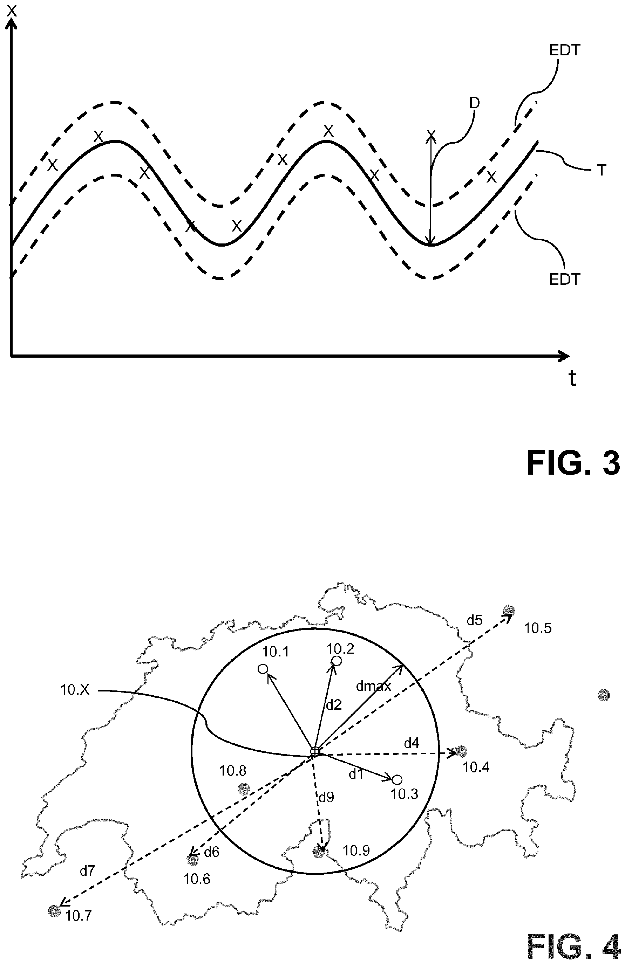

Figure 3 illustrates an example of how the actual usage data X is processed in view of the usual demand level T in order to determine the deviation D of the actual usage data X from the usual demand level T, respectively to detect deviations D which exceed the exceptional deviation threshold EDT. The illustrated fluctuation of the usual demand level T could be for example due to different levels of activities in the laboratory 10 in different times of day (day shift vs. night shift); weekdays vs. weekend; winter (flu season) vs. summer, etc. The aim of comparing the actual usage data X with the usual demand level T is therefore to detect deviations which exceed/contravene the expected pattern/trend as such can be an indication of a situation (e.g. epidemic) which leads to increased demand in the target laboratory(s) 10.X in proximity.

Turning back to figure 2, if the deviation D is above the exceptional deviation threshold EDT, an alert triggering factor F is calculated.

This alert triggering factor F is calculated for each and every deviation D that exceeds an exceptional deviation threshold EDT. Herein, each and every deviation D comprises multiple deviations D originating from the same triggering laboratory 10.1-10.n as well as deviations D originating from the various triggering laboratories 10.1-10.n.

The alert triggering factor F is a function of one or more of: the usual demand level T, the deviation D, and the proximity P of the triggering laboratory 10.1-10.n and the target laboratory 10.X.

According to particular embodiments of the disclosed system/ method, the alert triggering factor F is a weighted function, wherein the higher the usual demand level T the higher its alert-triggering weighting; and/or the higher the deviation D from the exceptional deviation threshold EDT, the higher its alert-triggering weighting.

According to a first embodiment, according to a further embodiment, the alert triggering factor F is calculated by the control unit 20 based on usual demand level T of the triggering laboratories, actual usage data X of the triggering laboratories received via the communication network 40 and the proximity P of the triggering laboratory 10.1-10.n and the target laboratory 10.X.

Alternatively or additionally, the alert triggering factor F is calculated by the respective triggering laboratory 10.1-10.n itself. In such embodiments, the triggering laboratory 10.1-10.n is configured to transmit the alert triggering factor F to the control unit 20 via the communication network 40.

Following the calculation of the alert triggering factor F of each deviation D that exceeds an exceptional deviation threshold EDT, each alert triggering factor F corresponding to the triggering laboratories 10.1-10.n in the proximity P of the target laboratory 10.X are aggregated.

By aggregating only the alert triggering factor(s) F of the triggering laboratories 10.1-10.n in the proximity P of the target laboratory 10.X, the disclosed system / method aims at filtering out alert triggers which due to their non-proximity are not relevant to the particular target laboratory 10.X. In other words, the aggregation of select alert triggering factor(s) F is tailored to the specific target laboratory 10.X.

According to embodiments of the disclosed system/method, the proximity P is a function, in particular a weighted function of one or a combination of more of the following parameters:

- a geographical distance d1-dn between the triggering laboratory 10.1-10.n and the target laboratory 10.X;
- a travelling distance dt (considering a certain transport infrastructure, e.g roads or railway) between the triggering laboratory 10.1-10.n and the target laboratory 10.X;
- a travelling time tt between the triggering laboratory 10.1-10.n and the target laboratory 10.X;
- a travelling frequency tf between the triggering laboratory 10.1-10.n and the target laboratory 10.X;
- a selected geographical and/or political area A.

Figure 4 illustrates an example of geographical distribution of the plurality of laboratories 10 comprising the triggering laboratories 10.1-10.8 and one target laboratory 10.X. In the embodiment illustrated, the proximity P is a function, wherein the geographical distance d1-dn between the triggering laboratory 10.1-10.n and the target laboratory 10.X is at least one of the parameters mentioned above. For example, the further the triggering laboratory 10.1-10.n the lower its contribution to the alert triggering factor F.

However, the geographical distance d1-dn between the triggering laboratory 10.1-10.n is not the only parameter, as exemplified by the laboratories 10.8 respectively 10.9, which - while closer to the target laboratory 10.X than the maximum geographical distance dmax, are not considered for the aggregated alert triggering factor $F_{tot}$. For example because laboratory 10.8 did not show a deviation D which exceeded the exceptional deviation threshold EDT, while laboratory 10.9 is in a different political area (different country) which might have different regulations on certain laboratory tests.

According to embodiments of the disclosed system/method, the weighted proximity function P has one or more of the following weights:

- a known infectious disease spread pattern overlapping the triggering laboratory 10.1-10.n and the target laboratory 10.X;
- a known major event with a known and/or expected elevated infectious disease risk level with known and/or suspected common participants from the geographical area of the triggering laboratory 10.1-10.n and the target laboratory 10.X.

As a next step, the aggregation $F_{tot}$ of each alert triggering factor F corresponding to each triggering laboratory 10.1-10.n in the proximity P of the target laboratory 10.X is compared to an alert threshold AT.

$$\mathbf{F_{tot}} = \sum_{i=1}^{n} \mathbf{Fi} > \mathbf{AT} \ ?$$

If the aggregation $F_{tot}$ exceeds to the alert threshold AT, the target laboratory 10.X is alerted, in particular via the communication network 40.

Alerting the target laboratory 10.X comprises - but is not limited to - transmitting an alert signal to the target laboratory 10.X which causes a user interface thereof to emit an audible and/or visual indication of the alert, such as a beeping sound drawing attention to an alert message displayed on said user interface of the target laboratory 10.X. Alternatively or additionally, transmitting an alert signal to the target laboratory 10.X comprises sending an electronic notification such as an email to an email account associated with the target laboratory 10.X, respectively with a user responsible for the consumable inventory associated with the target laboratory 10.X. Alternatively or additionally, transmitting an alert signal to the target laboratory 10.X comprises interfacing with an inventory management system of thereof, the alert causing the entry corresponding to the particular consumable to be flagged.

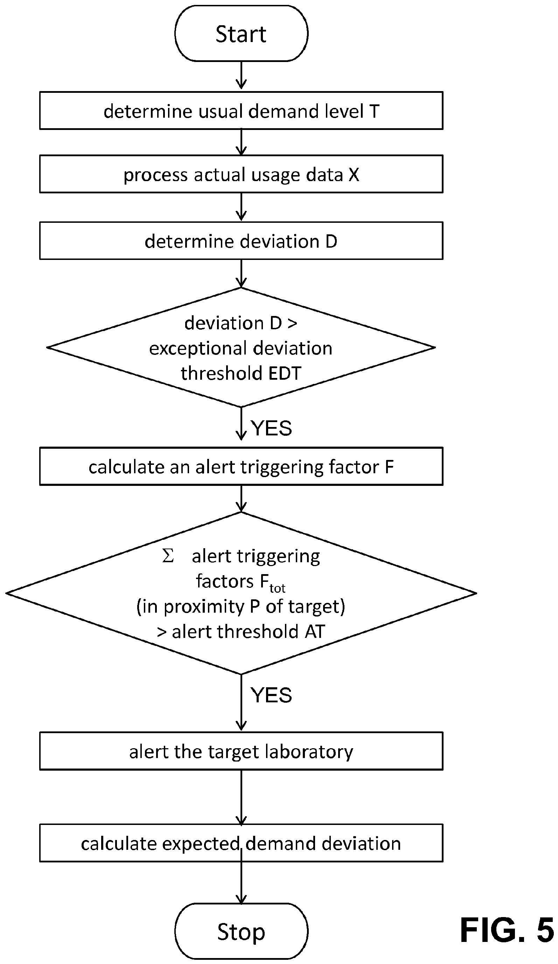

Figure 5 shows a flowchart of embodiments of the disclosed system/method further comprising the calculation of an expected demand deviation of the consumable for the target laboratory 10.X.

According to embodiments of the disclosed system/method, the expected demand deviation is calculated as a function of the usual demand level T of the target laboratory 10.X and/or a function of each alert triggering factor F corresponding to each triggering laboratory 10.1-10.n in the proximity P of the target laboratory 10.X and/or a function of the deviation D of each triggering lab. For example a large deviation D of a consumable at a triggering laboratory 10.1-10.n in the close proximity P (e.g. geographical closeness) will have a larger effect than a comparatively small deviation D of the same consumable at a triggering laboratory 10.1-10.n far from the target laboratory 10.X. As a further example, the same aggregated alert triggering factor $F_{tot}$ would have a greater effect on the expected demand deviation of a large target laboratory 10.X with a high usual demand level T of the corresponding consumable than a small target laboratory 10.X with a low usual demand level T of the same. Also demand deviation at a specialist laboratory would have a reduced effect on a routine laboratory.

According to embodiments of the disclosed system/method, alerts and/or the alert triggering factor F are specific and/or configurable for particular analytic test(s) or type of test(s), such as for example an analytic test for infectious diseases which are greatly exposed to fluctuations (exceptional deviations) and/or analytic tests which are time critical (e.g. in emergency testing environments).

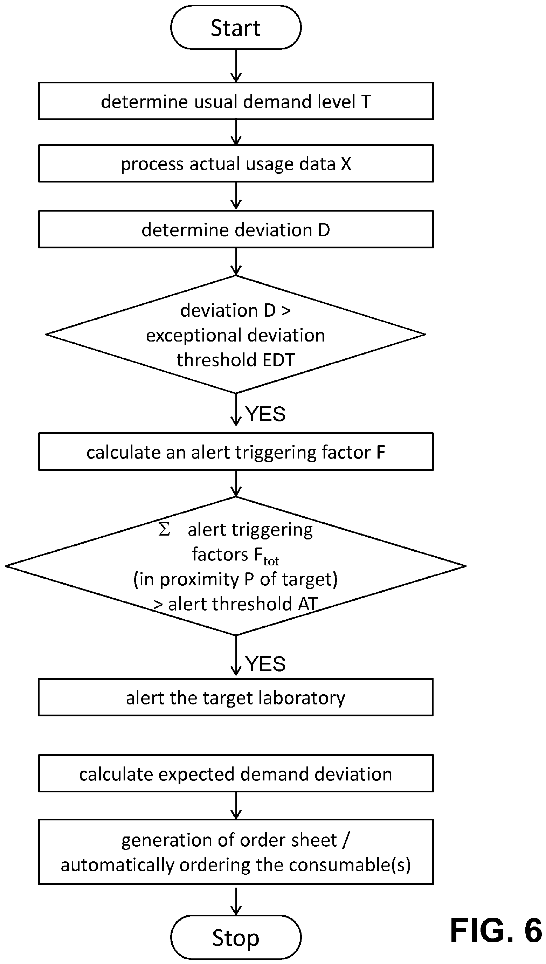

Figure 6 shows a flowchart of embodiments of the disclosed system/method further comprising generation of an order sheet of the corresponding consumable based on the expected demand deviation of the consumable for the target laboratory 10.X. Furthermore, the order sheet is also generated based on the actual inventory of the consumable in the target laboratory 10.X. The order sheet may be a conventional list of consumables to be ordered. Alternatively or additionally, the order sheet may be an electronic record to be processed by an inventory management system.

Hence, according to embodiments of the disclosed system/method, the corresponding consumable is automatically ordered based on the order sheet for target laboratory 10.X.

The order sheet may be a conventional list of consumables to be ordered. Alternatively or additionally, the order sheet may be an electronic record to be processed by an inventory management system.

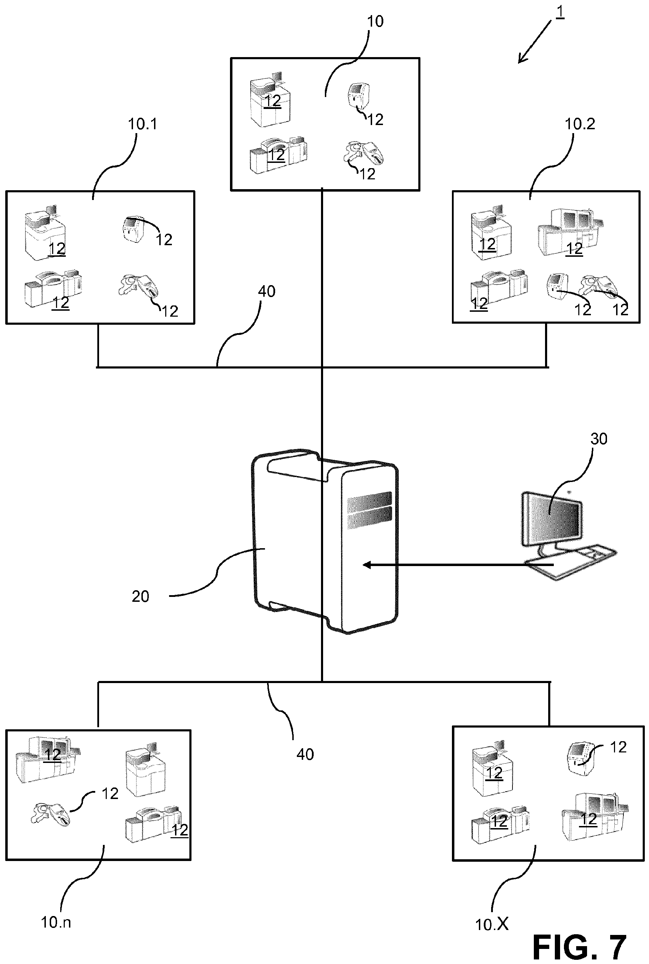

Figure 7 shows a further embodiment of the inventory alert system 1, further comprising an input device 30 communicatively connected to the control unit 20, either directly or via the communication network 40. The input device 30 may comprise - but is not limited to:

-   a personal computer, such as a desktop or laptop computer, having a user interface, i.e a piece of software and/or hardware for interactions between an operator and a machine, including but not limited to a graphical user interface for receiving an input from an operator and also to provide feedback and convey information thereto;
-   a portable computing device, i.e. a mobile/ portable electronic appliance having an interface for communicating with a server computer via a communication network, in particular a handheld battery powered mobile appliance, such as a mobile phone, a smart phone, a personal digital assistant (PDA) or another electronic appliance having an interface (wired and/or wireless) for establishing a communication link with a server computer such as over a wireless digital cellular telecommunications network or another communication channel;
-   a web interface accessible using a web browser for receiving an input from an operator.

Furthermore, the control unit 20 of the embodiments depicted on figure 7 is configured to receive an input from the input device 30. The input received from the input device is indicative of an accuracy of the calculation of an expected demand deviation. In an embodiment, this input which is indicative of the accuracy of the calculation is based on a comparison of previously calculated with the actually experienced deviations of consumable demand/usage by target laboratories 10.X. In a further embodiment, this input which is indicative of the accuracy of the calculation is based on an expert review of expected demand deviations.

Based on this input indicative of an accuracy of the calculation of an expected demand deviation, the control unit 20 is configured to adjust the exceptional deviation threshold EDT and/or the alert threshold AT and/or parameters of the function for calculation of the alert triggering factor F and/or parameters of the function for calculation of the expected demand deviation. By said adjustment the disclosed system/method is capable of improving sensitivity and specificity of its alerts as well as the accuracy of the expected demand deviation the longer the system/method is deployed, therefore providing even better and better optimization of the inventories of consumables of the corresponding laboratories 10.1-10.n.

For example if a comparison shows that the expected demand deviation has been predicted to be far off from the actual deviation reflected by following actual usage data X, an expert review (either by machine learning algorithms or manually) will be triggered to determine which factor, threshold and/or parameter needs to be adjusted to ensure better predictions in the future.

In summary, the disclosed system/method is advantageous as it allows multiple levels of configurability and adaptability:

- by means of the exceptional deviation threshold EDT, normal consumable demand fluctuations are filtered out in a manner specific to each triggering laboratory 10.1-10.n;
- by means of the proximity function P - and its weighting(s), the relevance (or influence) of each triggering laboratory 10.1-10.n is fine tuned to the specific target laboratory 10.X; and
- by setting an appropriate alert threshold AT of aggregated triggering factors $F_{tot}$, false triggers due to alert trigger factors of low relevance can be filtered out.

In other words, the disclosed system/method is adaptable to the specifics of each triggering laboratory 10.1-10.n; of each target laboratory 10.X and of the entire system of a plurality of laboratories 10.

Further disclosed and proposed is a computer program including computer-executable instructions for performing the disclosed method in one or more of the embodiments enclosed herein when the program is executed on a computer or computer network. Specifically, the computer program may be stored on a computer-readable data carrier. Thus, specifically, one, more than one or even all of method steps may be performed by using a computer or a computer network, preferably by using a computer program.

Further disclosed and proposed is a computer program product having program code means, in order to perform the disclosed method in one or more of the embodiments enclosed herein when the program is executed on a computer or computer network. Specifically, the program code means may be stored on a computer-readable data carrier. The product may generally exist in an arbitrary format, such as in a paper format, or on a computer-readable data carrier. Specifically, the computer program product may be distributed over a data network.

Further disclosed and proposed is a data carrier having a data structure stored thereon, which, after loading into a computer or computer network, such as into a working memory or main memory of the computer or computer network, may execute the disclosed method according to one or more of the embodiments disclosed herein.

As used herein, a computer program product refers to the program as a tradable product.

Further disclosed and proposed is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

It will be understood that many variations could be adopted based on the specific structure hereinbefore described without departing from the scope of the invention as defined in the following claims.

## REFERENCE SIGNS LIST

| | |
|---|---|
| inventory alert system | 1 |
| laboratories | 10 |
| triggering laboratory | 10.1-10.n |
| target laboratory | 10.X |
| laboratory instrument | 12 |
| control unit | 20 |
| input device | 30 |
| communication network | 40 |
| usual demand level | T |
| actual usage data | X |
| deviation (of the actual usage data) | D |
| exceptional deviation threshold | EDT |
| alert triggering factor | F |
| aggregated alert triggering factor | Ftot |
| alert threshold | AT |
| proximity | P |
| geographical distance | d |
| a travelling distance | dt |
| travelling time | t |
| travelling frequency | tf |

(continued)

| geographical and/or political area | A |
|---|---|

**Claims**

1.  Inventory alert system (1) for laboratories, comprising:

    - a plurality of laboratories (10) comprising at least one triggering laboratory (10.1-10.n) and at least one target laboratory (10.X), each laboratory (10) comprising at least one laboratory instrument (12) configured to carry out at least one processing step of a biological sample, a consumable being required to carry out the processing step;
    - a control unit (20) communicatively connected to each of the plurality of laboratories (10) by means of a communication network (40),

    **characterized in that**:

    - determine a usual demand level T of the consumable required to carry out the processing step by the corresponding triggering laboratory (10.1-10.n);
    - process actual usage data X of the consumable required to carry out the processing step by the corresponding triggering laboratory (10.1-10.n);
    - determine a deviation D of the actual usage data X from the usual demand level T of the corresponding triggering laboratory (10.1-10.n);
    - calculate an alert triggering factor F of each deviation D that exceeds an exceptional deviation threshold EDT;
    - alert the target laboratory (10.X) when an aggregation $F_{tot}$ of each alert triggering factor F corresponding to each triggering laboratory (10.1-10.n) in the proximity P of the target laboratory (10.X) exceeds an alert threshold AT.

2.  Inventory alert system (1) according claim 1, wherein the usual demand level T of the consumable required to carry out the processing step of a biological sample is determined based on an average and/or median number of conducted and/or ordered processing step(s) per defined period corresponding to the respective triggering laboratory (10.1-10.n) and/or determined based on historical data comprising one or more of: a seasonal variation; a schedule-based variation; an event-linked variation and a trend associated variation; geographically influenced variation.

3.  Inventory alert system (1) according to one of the preceding claims, wherein:

    - the alert triggering factor F is a function of one or more of: the usual demand level T, the deviation D, the proximity P of the triggering laboratory (10.1-10.n) and the target laboratory (10.X); and/or
    - the proximity P is a function, in particular a weighted function of one or a combination of more of the following parameters:

        - a geographical distance d1-dn between the triggering laboratory (10.1-10.n) and the target laboratory (10.X);
        - a travelling distance dt between the triggering laboratory (10.1-10.n) and the target laboratory (10.X);
        - a travelling time tt between the triggering laboratory (10.1-10.n) and the target laboratory (10.X);
        - a travelling frequency tf between the triggering laboratory (10.1-10.n) and the target laboratory (10.X)
        - a selected geographical and/or political area A;

    - the proximity function P having one or more of the following weights:

        - a known infectious disease spread pattern overlapping the triggering laboratory (10.1-10.n) and the target laboratory (10.X);
        - a known major event with a known and/or expected elevated infectious disease risk level with known and/or suspected common participants from the geographical area of the triggering laboratory (10.1-10.n) and the target laboratory (10.X).

4.  Inventory alert system (1) according to one of the preceding claims, wherein alerting the target laboratory (10.X) comprises calculation of an expected demand deviation of the consumable for the target laboratory (10.X), the expected demand deviation being a function of the usual demand level T of the target laboratory (10.X) and/or a function of each alert triggering factor F corresponding to each triggering laboratory (10.1-10.n) in the proximity P of the target laboratory (10.X) and/or a function of the deviation D of each triggering lab.

5.  Inventory alert system (1) according to claim 4, wherein alerting the target laboratory (10.X) comprises generation of an order sheet of the corresponding consumable based on the expected demand deviation of the consumable for the target laboratory (10.X) and the actual inventory of the consumable in the target laboratory (10.X) and/or automatically ordering the corresponding consumable based on the order sheet for target laboratory (10.X).

**6.** Inventory alert system (1) according to one of the claims claim 5, further comprising an input device (30), wherein the control unit (20) is configured to:

- receive an input from the input device (30), the input being indicative of an accuracy of the calculation of an expected demand deviation;
- adjust the exceptional deviation threshold EDT and/or the alert threshold AT and/or parameters of the function for calculation of the alert triggering factor F and/or parameters of the function for calculation of the expected demand deviation based on said input.

**7.** Inventory alert system (1) according to one of the preceding claims, wherein the at least one laboratory instrument (12) comprises at least one analytical instrument and wherein the at least one processing step of a biological sample comprises at least one diagnostic test of the biological sample for obtaining a measurement value from biological sample.

**8.** A computer implemented method for alerting laboratories comprising the steps:

- communicatively connecting a plurality of laboratories (10) to a control unit (20), wherein the plurality of laboratories (10) comprise at least one triggering laboratory (10.1-10.n) and at least one target laboratory (10.X), each laboratory (10) comprising at least one laboratory instrument (12) configured to carry out at least one processing step of a biological sample, a consumable being required to carry out the processing step;
- determining a usual demand level T of the consumable required to carry out the processing step by the corresponding triggering laboratory (10.1-10.n);
- processing actual usage data X of the consumable required to carry out the processing step by the corresponding triggering laboratory (10.1-10.n);
- determining a deviation D of the actual usage data X from the usual demand level T of the corresponding triggering laboratory (10.1-10.n);
- calculating an alert triggering factor F of each deviation D that exceeds an exceptional deviation threshold EDT;
- the control unit (20) alerting the target laboratory (10.X) when an aggregation $F_{tot}$ of each alert triggering factor F corresponding to each triggering laboratory (10.1-10.n) in the proximity P of the target laboratory (10.X) exceeds an alert threshold AT.

**9.** Method for alerting laboratories according to claim 8, wherein the usual demand level T of the consumable required to carry out the processing step is determined based on an average and/or median number of conducted and/or ordered processing step(s) per defined period corresponding to the respective triggering laboratory (10.1-10.n) and/or determined based on historical data comprising one or more of: a seasonal variation; a schedule-based variation; an event-linked variation and a trend associated variation; geographically influenced variation.

**10.** Method for alerting laboratories according to claim 8 or 9, wherein:

- the alert triggering factor F is a function of one or more of: the usual demand level T, the deviation D, and the proximity P of the triggering laboratory (10.1-10.n) and the target laboratory (10.X); and/or
- the proximity P is a function, in particular a weighted function of one or a combination of more of the following parameters:

    - a geographical distance d1-dn between the triggering laboratory (10.1-10.n) and the target laboratory (10.X);
    - a travelling distance dt between the triggering laboratory (10.1-10.n) and the target laboratory (10.X);
    - a travelling time tt between the triggering laboratory (10.1-10.n) and the target laboratory (10.X);
    - a travelling frequency tf between the triggering laboratory (10.1-10.n) and the target laboratory (10.X)
    - a selected geographical and/or political area A;

- the proximity function P having one or more of the following weights:

    - a known infectious disease spread pattern overlapping the triggering laboratory (10.1-10.n) and the target laboratory (10.X);
    - a known major event with a known and/or expected elevated infectious disease risk level with known and/or suspected common participants from the geographical area of the triggering laboratory (10.1-10.n) and the target laboratory (10.X).

**11.** Method for alerting laboratories according to one of the claims 8 to 10, further comprising the step of calculating an expected demand deviation of the consumable for the target laboratory (10.X), the expected demand deviation being a function of the usual demand level T of the target laboratory (10.X) and/or a function of each alert triggering factor F corresponding to each triggering laboratory (10.1-10.n)

in the proximity P of the target laboratory (10.X) and/or a function of the deviation D of each triggering lab.

12. Method for alerting laboratories according to claim 11, further comprising the step of generating an order sheet of the corresponding consumable based on the expected demand deviation of the consumable for the target laboratory (10.X) and the actual inventory of the consumable in the target laboratory (10.X) and/or automatically ordering the corresponding consumable based on the order sheet for target laboratory (10.X).

13. Method for alerting laboratories according to one of the claims 8 to 12, further comprising the steps of:

- receiving an input from an input device (30), the input being indicative of an accuracy of the calculation of an expected demand deviation;
- adjusting the exceptional deviation threshold EDT and/or the alert threshold AT and/or parameters of the function for calculation of the alert triggering factor F and/or parameters of the function for calculation of the expected demand deviation based on said input.

14. Method for alerting laboratories according to one of the claims 8 to 13, wherein the at least one laboratory instrument (12) comprises at least one analytical instrument and wherein the at least one processing step of a biological sample comprises at least one diagnostic test of the biological sample for obtaining a measurement value from biological sample.

15. Computer program product having program code means, in order to perform the method according to one of the claims 8 to 14 when the program is executed on a computer or computer network.

FIG. 1

Start

↓

determine usual demand level T

↓

process actual usage data X

↓

determine deviation D

↓

deviation D >
exceptional deviation
threshold EDT

YES

↓

calculate an alert triggering factor F

↓

$\Sigma$  alert triggering
factors $F_{tot}$
(in proximity P of target)
> alert threshold AT

YES

↓

alert the target laboratory

↓

Stop

**FIG. 2**

**FIG. 3**

**FIG. 4**

```
                    ┌─────────────┐
                    │    Start    │
                    └──────┬──────┘
                           │
                           ▼
        ┌──────────────────────────────────────┐
        │     determine usual demand level T    │
        └──────────────────┬───────────────────┘
                           │
                           ▼
        ┌──────────────────────────────────────┐
        │       process actual usage data X     │
        └──────────────────┬───────────────────┘
                           │
                           ▼
        ┌──────────────────────────────────────┐
        │         determine deviation D         │
        └──────────────────┬───────────────────┘
                           │
                           ▼
```

deviation D >
exceptional deviation
threshold EDT

YES

calculate an alert triggering factor F

$\Sigma$ alert triggering
factors $F_{tot}$
(in proximity P of target)
> alert threshold AT

YES

alert the target laboratory

calculate expected demand deviation

```
                    ┌─────────────┐
                    │    Stop     │
                    └─────────────┘
```

**FIG. 5**

```
                            ┌──────────────┐
                            │    Start     │
                            └──────┬───────┘
                                   │
                                   ▼
              ┌────────────────────────────────────────┐
              │    determine usual demand level T       │
              └────────────────────┬───────────────────┘
                                   │
                                   ▼
              ┌────────────────────────────────────────┐
              │       process actual usage data X       │
              └────────────────────┬───────────────────┘
                                   │
                                   ▼
              ┌────────────────────────────────────────┐
              │          determine deviation D          │
              └────────────────────┬───────────────────┘
                                   │
                                   ▼
                           deviation D >
                       exceptional deviation
                          threshold EDT
                                   │ YES
                                   ▼
              ┌────────────────────────────────────────┐
              │   calculate an alert triggering factor F │
              └────────────────────┬───────────────────┘
                                   │
                                   ▼
                          Σ  alert triggering
                            factors F_tot
                        (in proximity P of target)
                          ≥ alert threshold AT
                                   │ YES
                                   ▼
              ┌────────────────────────────────────────┐
              │        alert the target laboratory       │
              └──────────────────────────────────────────┘

              ┌────────────────────────────────────────┐
              │      calculate expected demand deviation │
              └────────────────────┬───────────────────┘
                                   │
                                   ▼
              ┌────────────────────────────────────────┐
              │       generation of order sheet /        │
              │   automatically ordering the consumable(s)│
              └────────────────────┬───────────────────┘
                                   │
                                   ▼
                            ┌──────────────┐
                            │    Stop      │
                            └──────────────┘
```

**FIG. 6**

FIG. 7

Europäisches
Patentamt

European
Patent Office

Office européen
des brevets

# EUROPEAN SEARCH REPORT

Application Number

EP 16 19 7229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/246215 A1 (POSTMA STEPHEN J [US] ET AL) 6 October 2011 (2011-10-06) * paragraphs [0024] - [0029], [0045] - [0048]; figures 1,4,5 * ----- | 1-15 | INV. G06Q10/08 G01N35/00 |
| X | US 2009/134978 A1 (IMAI NAOYA [JP]) 28 May 2009 (2009-05-28) * paragraphs [0012], [0032]; figure 1 * ----- | 1-15 | |

TECHNICAL FIELDS
SEARCHED (IPC)

G06Q
G01N

The present search report has been drawn up for all claims

| Place of search | Date of completion of the search | Examiner |
|---|---|---|
| The Hague | 4 April 2017 | van Lith, Joris |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 19 7229

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2017

| Patent document cited in search report | | | Publication date | Patent family member(s) | | Publication date |
|---|---|---|---|---|---|---|
| US 2011246215 | A1 | | 06-10-2011 | CN 102207993 A | | 05-10-2011 |
| | | | | EP 2381379 A2 | | 26-10-2011 |
| | | | | JP 5739187 B2 | | 24-06-2015 |
| | | | | JP 5931247 B2 | | 08-06-2016 |
| | | | | JP 6030183 B2 | | 24-11-2016 |
| | | | | JP 2011209275 A | | 20-10-2011 |
| | | | | JP 2015163896 A | | 10-09-2015 |
| | | | | JP 2015180878 A | | 15-10-2015 |
| | | | | US 2011246215 A1 | | 06-10-2011 |
| US 2009134978 | A1 | | 28-05-2009 | CN 101448723 A | | 03-06-2009 |
| | | | | EP 2022737 A1 | | 11-02-2009 |
| | | | | JP 5199548 B2 | | 15-05-2013 |
| | | | | JP 2007314327 A | | 06-12-2007 |
| | | | | US 2009134978 A1 | | 28-05-2009 |
| | | | | WO 2007138827 A1 | | 06-12-2007 |

EPO FORM P0459

For more details about this annex : see Official Journal of the European Patent Office, No. 12/82